# EUROPÄISCHE PATENTANMELDUNG

(11) **EP 4 530 619 A1**
(43) Veröffentlichungstag der Anmeldung: **02.04.2025**
(21) Anmeldenummer: 24165726.1
(22) Anmeldetag: 24.03.2024
(51) Int. Cl.: G01N 25/72, G01N 33/44, B05C 5/02, G05B 19/418, B05C 11/10

(54) **VERFAHREN ZUR SOFTWAREBASIERTEN PRÜFUNG EINER ZU EINER DICHTUNG ERHÄRTENDEN MATERIALMISCHUNG**

(30) Priorität: 27.09.2023 LU 505198
(71) Anmelder: WILO SE, 44263 Dortmund (DE)
(72) Erfinder: Berger, Christian, 44263 Dortmund (DE); Gebhardt, Alexander, 44263 Dortmund (DE); Verstege, Wiebke, 44263 Dortmund (DE); Erhart, Alexander, 44263 Dortmund (DE); Friesen, Witali, 44263 Dortmund (DE)
(74) Vertreter: Cohausz Hannig Borkowski Wißgott

(57) **Zusammenfassung**

Die Erfindung betrifft ein Verfahren zur softwarebasierten Prüfung einer entlang eines Applikationspfades (9b) spritztechnisch an einem Bauteil (3), insbesondere an einem Elektronikgehäuse eines Pumpenaggregats, applizierten und zu einer Dichtung erhärtenden Materialmischung (8) aus wenigstens einer ersten und einer zweiten Komponente, die unmittelbar vor oder während des Applizierens in einem änderbar voreingestellten Mischungsverhältnis gemischt werden und dabei exotherm miteinander reagieren, **dadurch gekennzeichnet, dass** während der exothermen Reaktion mit einer Wärmebildkamera (12) wenigstens eine thermografische Aufnahme (14) der applizierten Materialmischung (8) erstellt und anschließend softwarebasiert daraufhin ausgewertet wird, ob die Temperatur der Materialmischung (8) entlang des Applikationspfades (9b) wenigstens eine Referenztemperatur über- oder unterschreitet, und/oder ob ein für die Applikation der Materialmischung vorgesehener Soll-Applikationspfad (9a) eingehalten wird oder ist.

## Beschreibung

Die Erfindung betrifft ein Verfahren zur softwarebasierten Prüfung einer entlang eines Applikationspfades spritztechnisch an einem Bauteil, insbesondere an einem Elektronikgehäuse eines Pumpenaggregats, applizierten und zu einer Dichtung erhärtenden Materialmischung aus wenigstens einer ersten und einer zweiten Komponente, die unmittelbar vor oder während des Applizierens in einem änderbar voreingestellten Mischungsverhältnis gemischt werden und dabei exotherm miteinander reagieren.

Dichtungen dienen bekannterweise der Abdichtung von Raumbereichen gegeneinander, so dass das Eindringen von Fremdstoffen, z.B. von Partikeln oder Feuchtigkeit aus dem einen Raumbereich in den anderen Raumbereich verhindert wird. Ein Beispiel hierfür ist die Abdichtung des Innenraums eines Elektronikgehäuses gegenüber der Außenwelt. Das Elektronikgehäuse besteht aus einem Gehäuseunterteil und einem Gehäuseoberteil, die gemeinsam den eine Elektronik aufnehmenden Innenraum umschließen und mit Hilfe der Dichtung gegeneinander abgedichtet sind. Das Gehäuseunterteil und das Gehäuseoberteil sind als Bauteile zu betrachten. Die Dichtung hat sowohl zu dem einen als auch zu dem anderen Bauteil Kontakt.

Damit die Dichtung dauerhaft ortsfest ist, und nicht etwas bei der Montage oder einer Demontage der Bauteile verrutscht, mit denen sie Kontakt hat, und um die Anzahl zu montierender Teile zu reduzieren, ist es bekannt, Dichtungen an eines der Bauteile anzuspritzen, so dass ein stofflicher Verbund zwischen Dichtung und Bauteil besteht. Hierzu wird eine im Verarbeitungszustand pastöse Dichtungsmasse verwendet, die nach der Applikation an dem Bauteil unter Erreichung ihres elastisch flexiblen Endzustands erhärtet. Es sei an dieser Stelle angemerkt, dass der Begriff "Erhärten" im Sinne der vorliegenden Beschreibung nicht bedeutet, dass die Dichtungsmasse nach einer Aushärtezeit "hart" bzw. unverformbar wird. Vielmehr ist der Begriff so zu verstehen, dass die Dichtungsmasse von dem ursprünglich pastösen Verarbeitungszustand in einen Zustand elastischer Verformbarkeit übergeht, d.h. nach dem "Erhärten" unter einer Krafteinwirkung reversibel verformbar ist. Mit anderen Worten ist die Dichtungsmasse, mithin die dann vorliegende Dichtung, nach ihrem Erhärten derart elastisch flexibel, dass sie nach der Krafteinwirkung in ihren ursprünglichen Formzustand zurückkehrt.

Je nach gewünschten Materialeigenschaften der Dichtung können verschiedene Dichtungsmassen verwendet werden. Insbesondere ist es bekannt, Materialmischungen aus zwei oder mehr Komponenten zu verwenden, die gemeinsam die Dichtungsmasse bilden. Durch das gezielte Mischen der Komponenten, kann beispielsweise die Aushärtezeit der Dichtung, ihre Flexibilität im Endzustand, ihre Farbe, eine etwaige chemische Resistenz usw. beeinflusst werden.

Bei der Applizierung der Dichtungsmasse entlang eines Applikationspfades auf einem Bauteil können unterschiedliche Fehler auftreten. Insbesondere ist es herausfordernd, stets dieselbe Menge an Dichtungsmasse pro Zeiteinheit und Längeneinheit bei fortschreitender Relativbewegung zwischen der Düse der Spritzmaschine, aus der die Dichtungsmasse austritt, und dem Bauteil zu erreichen. Fehler können dabei eine zu große Menge oder eine zu kleine Menge an Dichtungsmasse, eine zu starke Abweichung vom Applikationspfad und ein falsches Mischungsverhältnis sein.

Jede auf ein Bauteil applizierte Dichtung wird oft noch manuell, d.h. durch eine prüfende Person, sowohl visuell als auch haptisch auf Fehler geprüft. Dieser schaut sich alle Bauteile an. Besonders nachteilig ist hierbei, dass eine genaue Prüfung erst nach dem Erhärten der Dichtung, d.h. nach einiger Zeit möglich ist. Wird dann ein Fehler erkannt, kann dieser nicht (mehr) korrigiert werden und das Bauteil ist Ausschuss. In der Praxis gilt dies dann für die gesamte hergestellte Serie von Bauteilen ebenso. Dies ist nicht nur wirtschaftlich ärgerlich, sondern auch mit Blick auf die Umwelt und Ressourcen bedenklich.

Es ist deshalb Aufgabe der vorliegenden Erfindung, die Prüfung von Bauteilen mit applizierter Dichtung zu automatisieren und im Herstellungsprozess zeitlich vorzuziehen, so dass etwaige Fehler frühzeitiger erkannt werden können und die Herstellung weiterer fehlerbehafteter Bauteile verhindert werden kann.

Diese Aufgabe wird durch ein Verfahren mit den Merkmalen des Anspruchs 1 gelöst, Vorteilhafte Weiterbildungen sind in den Unteransprüchen angegeben und werden nachfolgend erläutert.

Erfindungsgemäß wird ein Verfahren zur softwarebasierten Prüfung einer entlang eines Applikationspfades spritztechnisch an einem Bauteil, insbesondere an einem Elektronikgehäuse eines Pumpenaggregats, applizierten und zu einer Dichtung erhärtenden Materialmischung aus wenigstens einer ersten und einer zweiten Komponente vorgeschlagen, die unmittelbar vor oder während des Applizierens in einem änderbar voreingestellten Mischungsverhältnis gemischt werden und dabei exotherm miteinander reagieren, wobei während der exothermen Reaktion mit einer Wärmebildkamera wenigstens eine thermografische Aufnahme (Bild) der applizierten Materialmischung erstellt und anschließend softwarebasiert daraufhin ausgewertet wird, ob die Temperatur der Materialmischung entlang des Applikationspfades wenigstens eine Referenztemperatur über- oder unterschreitet, und/oder ob ein für die Applikation der Materialmischung vorgesehener Soll-Applikationspfad (Ideallinie) eingehalten wird oder ist.

Der Kern der Erfindung besteht darin, die Reaktionswärme der Materialmischung auszunutzen, die infolge des Kontakts der ersten und der zweiten Komponente entsteht, um eine frühzeitige Aussage über die ordnungsgemäße Applikation der Materialmischung auf dem Bauteil treffen zu können. In der thermografischen Aufnahme ist die Temperatur der Materialmischung entlang des Applikationspfades sichtbar und kann folglich ausgewertet werden. Genauer gesagt, ist in der thermografischen Aufnahme jedem aufgenommenen Bildpunkt eine Temperatur zugeordnet. Anhand dieser Bildpunkttemperaturen kann festgestellt werden, ob auf dem Applikationspfad lokal eine zu geringe Temperatur oder eine zu hohe Temperatur, oder aber eine zu große Abweichung vom gewünschten Soll-Applikationspfad vorliegt. Eine zu geringe oder zu hohe Temperatur lässt ferner auf ein fehlerhaftes Mischungsverhältnis bzw. Dosierung der ersten und zweiten Komponente schließen. Somit kann ein Rückschluss auf die ordnungsgemäße Funktion der Dosiereinrichtung der Spritzmaschine getroffen werden, mit der die Materialmischung appliziert wird. Die manuelle Prüfung der Bauteile wird durch eine softwaretechnische Lösung substituiert, indem die wenigstens eine mit Hilfe der Wärmebildkamera erstellte thermografische Aufnahme softwaretechnische ausgewertet wird.

Je nach den gewünschten Materialeigenschaften der Dichtung können verschiedene Dichtungsmassenkomponenten verwendet werden. Beispielsweise können die erste und zweite Komponente derart gewählt sein, dass sie infolge ihrer Mischung aufschäumen. Mit anderen Worten wird eine aufschäumende Dichtungsmasse verwendet, deren Volumen sich während des Erhärtens unter Gasbildung erhöht, so dass anschließend eine weiche, porige Schaumdichtung vorliegt. Diese hat den Vorteil, einen besonders breitflächigen Kontakt zum Verbindungspartner des Bauteils bei gleichzeitig guter Dichtungswirkung und vergleichsweise geringer Rückstellkraft herzustellen, so dass sie das Bauteil und seinen Verbindungspartner nur leicht voneinander wegdrückt.

Die Verwendung einer aufschäumenden Materialmischung stellt in der Praxis eine besondere Herausforderung dar, weil sich ihr Endvolumen für eine visuelle Prüfung erst nach einer gewissen Verzögerungszeit ergibt. Dieses Problem wird bei dem erfindungsgemäßen Verfahren jedoch behoben, da die Temperatur der Materialmischung bereits unmittelbar nach bzw. wenige Sekunden nach der Applizierung eine Aussage darüber zulässt, ob das gewünschte Endvolumen erreicht oder auch überschritten wird.

Die Auswertung der thermografischen Aufnahme oder Aufnahmen kann dadurch erfolgen, dass sie mit wenigstens einem Referenzdatensatz verglichen wird, der aus Referenzbildern von Bauteilen mit fehlerhaft applizierter Materialmischung und/ oder mit fehlerfrei, oder ideal, applizierter Materialmischung erstellt worden ist. Der Referenzbilddatensatz kann also entweder nur aus thermografischen Aufnahmen erstellt sein, in denen die Applikation der Materialmischung in Ordnung ist, oder nur aus thermografischen Aufnahmen erstellt sein, in denen die Applikation der Materialmischung nicht in Ordnung ist, oder aus thermografischen Aufnahme beider Fälle erstellt sein. Der Vergleich kann pixelweise oder pixelclusterweise erfolgen. Der aus den Referenzbildern gebildete Referenzdatensatz stellt eine Art Wissensdatenbank dar, auf deren Basis die neu hergestellten Bauteile überprüft werden.

Beispielsweise kann ein Übereinstimmungs- oder Abweichungsgrad der thermografischen Aufnahme oder Aufnahmen zum Referenzdatensatz bestimmt werden. Anhand des Übereinstimmungs- oder Abweichungsgrades kann entschieden werden, ob ein hergestelltes Bauteil in Ordnung oder Ausschuss ist. Bevorzugt kann diese softwaretechnische Auswertung unter Verwendung künstlicher Intelligenz, d.h. mittels eines KI-Algorithmusses erfolgen. Wenn der Übereinstimmungsgrad einen Grenzwert unterschreitet oder der Abweichungsgrad einen Grenzwert überschreitet, kann eine Meldung ausgegeben werden, die Applikation der Materialmischung beendet und/ oder eine Korrekturmaßnahme eingeleitet werden.

Alternativ oder kumulativ können aus der thermografischen Aufnahme der Materialmischung entlang des Applikationspfades zuordenbare, lokal maximale Temperaturwerte bestimmt werden, die dann jeweils mit der wenigstens einen Referenztemperatur verglichen werden. Auch hier kann eine Meldung ausgegeben, die Applikation der Materialmischung beendet und/ oder eine Korrekturmaßnahme automatisch eingeleitet werden, wenn die Referenztemperatur an wenigstens einer Stelle des Applikationspfades nicht erreicht oder überschritten wird. Wenn der Fehlertyp Mischverhältnis als "nicht korrekt" oder "nicht in Ordnung" identifiziert wird, kann auch die Empfehlung ausgegeben werden, dass die Anlage eine Reinigung durchführen soll. Alternativ kann automatisch eine Reinigung an der Spritzmaschine ausgelöst werden. Durch das Reinigen der Anlage werden mittels einer Spülung die Leitungen sowie der Spritzkopf in einen Ursprungszustand versetzt. Dadurch werden eventuelle Luftblasen, die zur einem inkorrekten Mischverhältnis führen können, und/ oder sonstige inneren Anhaftungen, die das Applizieren eines korrekten voreingestellten Volumenstroms unterbinden oder gar die Materialmischung entmischen könnten, aus der Anlage eliminiert. Darüber hinaus kann auch ein Hinweis auf eine defekte Fördertechnik der Anlage wenigstens einer an der Mischung beteiligter Materialkomponenten ausgegebenen werden. In diesem engeren Fehlerrahmen würde dies eine selbstständige Regelung der Spritzmaschine bedeuten.

Die Referenztemperatur kann eine Mindesttemperatur sein, wobei die Meldung ausgegeben, die Applikation beendet und/ oder die Korrekturmaßnahme eingeleitet wird, wenn die Referenztemperatur nicht erreicht wird. In diesem Fall ist die exotherme Reaktion offensichtlich zu gering, woraus sich schließen lässt, dass entweder eine zu geringe Menge der Materialmischung appliziert worden ist oder das Mischungsverhältnis zwischen der ersten und zweiten Komponente nicht korrekt eingestellt ist.

Alternativ kann die Referenztemperatur eine Maximaltemperatur sein, wobei die Meldung ausgegeben, die Applikation beendet und/ oder die Korrekturmaßnahme eingeleitet wird, wenn der Referenzwert erreicht oder überschritten wird. In diesem Fall ist die exotherme Reaktion offensichtlich zu stark, woraus sich schließen lässt, dass entweder eine zu große Menge der Materialmischung appliziert worden ist oder das Mischungsverhältnis zwischen der ersten und zweiten Komponente nicht korrekt eingestellt ist.

Selbstverständlich kann die Temperatur der Materialmischung entlang des Applikationspfades sowohl mit einer ersten Referenztemperatur, die der genannten Mindesttemperatur entspricht, als auch mit einer zweiten Referenztemperatur verglichen werden, die der genannten Maximaltemperatur entspricht. Bei beiden Vergleichen kann dann die Meldung ausgegeben, die Applikation beendet und/ oder die Korrekturmaßnahme eingeleitet werden, wenn die erste Referenztemperatur nicht erreicht oder die zweite Referenztemperatur erreicht oder überschritten wird.

Vorzugsweise wird aus lokal maximalen Temperaturwerten die Lage und/ oder der Verlauf der Materialmischung innerhalb der thermografischen Aufnahme bestimmt, entlang welchem die lokal maximalen Temperaturwerten liegen. Der Verlauf der Materialmischung kann als Ist-Applikationspfad betrachtet werden. Anschließend kann oder können die Lage und/ oder der Verlauf der Materialmischung mit dem Soll-Applikationspfad verglichen werden, und eine Meldung ausgegeben, die Applikation beendet und/ oder die Korrekturmaßnahme eingeleitet werden, wenn die Lage und/ oder der Verlauf der Materialmischung an wenigstens einer Stelle um ein vorgegebenes Maß vom Soll-Applikationspfad abweicht.

In einer Ausführungsvariante erfasst die Wärmebildkamera die Materialmischung an einem ersten Ort des Applikationspfades, während die Materialmischung an einem zweiten Ort des Applikationspfades auf das Bauteil appliziert wird. Die thermografische Erfassung der Materialmischung erfolgt hier also während bzw. gleichzeitig mit der Applikation der Materialmischung. An die Erfassung schließt sich selbstverständlich die Auswertung der thermografischen Aufnahme an, so dass quasi eine "in situ"- oder "live" Prüfung des Applikationsgeschehens erreicht wird. Somit kann ein etwaiger Fehler schnell festgestellt und darauf reagiert werden.

Geografisch betrachtet, nimmt die Wärmebildkamera nur einen Teilabschnitt der applizierten Materialmischung auf, während die Materialmischung außerhalb dieses Teilabschnitts gerade erst entsteht. In dieser Ausführungsvariante kann die Wärmebildkamera Teil der Spritzmaschine sein. Sie kann gemeinsam mit der Düse der Spritzmaschine verfahren und/ oder einen festen Abstand zu ihr haben. Die Wärmebildkamera bewegt sich in diesem Fall ebenfalls relativ zum Bauteil. Insbesondere kann die Düse der Wärmebildkamera räumlich vorausgehen. Anders betrachtet, wird die Materialmischung von der Düse an einem ersten Ort des Soll-Applikationspfades auf das Bauteil appliziert, wobei sich dieser Ort aufgrund der Relativbewegung zwischen Wärmebildkamera und Bauteil anschließend in den Erfassungsbereich der Wärmebildkamera bewegt oder der Erfassungsbereich der Wärmebildkamera über den genannten Ort bewegt, so dass die thermografische Aufnahme des diesen Ort umfassenden Teilabschnitts erstellt werden und anschließend ausgewertet werden kann.

In einer anderen Ausführungsvariante erfasst die Wärmebildkamera das gesamte Bauteil nach der Applikation der Materialmischung. Mit anderen Worten nimmt die Wärmebildkamera die thermografische Aufnahme erst nach der vollständigen Applikation der Dichtung entlang des gesamten Applikationspfades auf. Insbesondere können auch mehrere hergestellte Bauteile, beispielsweise auf einem Tableau nebeneinanderliegend, gleichzeitig erfasst und ausgewertet werden, um die Effizienz der Prüfung zu steigern. In dieser Ausführungsvariante kann die Wärmebildkamera baulich und/ oder örtlich unabhängig von der Düse der Spritzmaschine sein.

Sofern das Bauteil einer Serie von Bauteilen entstammt, denen auf derselben Spritzmaschine die Materialmischung appliziert worden ist, ist es zur Fehlerdiagnose sinnvoll, wenn der Referenzdatensatz aus thermografischen Aufnahmen der ersten hergestellten Bauteile mit applizierter Materialmischung der Serie erstellt ist. Auf diese Weise kann eine allmähliche Verschlechterung der Qualität der Dichtung erkannt werden, insbesondere eine Veränderung des Mischungsverhältnisses (Dosierung), beispielsweise infolge einer zunehmenden Verstopfung eines Dosierkanals für eine der Materialkomponenten, und/oder eine zunehmende Abweichung (Drift) vom Soll-Applikationspfad.

Sinnvollerweise sind die thermografische Aufnahme der applizierten Materialmischung und die thermografischen Aufnahmen, aus denen der Referenzdatensatz erstellt wurde, zu im Wesentlichen gleichen Zeitpunkten oder innerhalb gleicher Zeitfenster nach der vollständigen Applizierung der Materialmischung auf das jeweilige Bauteil aufgenommen worden, insbesondere mit maximal wenigen Sekunden Unterschied zwischen der Aufnahme und dem Applikationsende. Der Hintergrund dieser Bedingung liegt darin, dass die Temperatur der Materialmischung mit der Zeit abnimmt, d.h. eine durch den Referenzdatensatz festgelegte, auf einen Ort des Applikationspfads bezogene Idealtemperatur zeitbezogen ist, mithin sich zeitlich ändert, so dass nur der Vergleich zeitlich korrelierender thermografsicher Aufnahmen eine korrekte Aussage über die Temperaturverhältnisse entlang des Applikationspfades ermöglicht.

Wie bereits in der Beschreibungseinleitung angemerkt, kann es sich bei dem Bauteil beispielsweise um ein erstes Gehäuseteil eines Elektronikgehäuses handeln, das bestimmungsgemäß in dichtendem Kontakt mit einem zweiten Gehäuseteil gelangt. Das erste Gehäuseteil kann kasten-, wannen- oder schalenförmig sein, wohingegen das zweite Gehäuseteil einen Deckel bilden kann. Die Gehäuseteile können miteinander verschraubt, verrastet oder verklemmt werden. Das erste Gehäuseteil kann, im Falle der Kastenform an den Stirnseiten der Seitenwände, eine Umfangskante aufweisen, auf die die Materialmischung zur Bildung der Dichtung appliziert bzw. aufgespritzt wird. Der Verlauf der Umfangskante bildet in diesem Fall den Applikationspfad. Damit die Materialmischung nach ihrer Applizierung nicht verläuft, kann die Umfangskante eine Nut aufweisen, in die die Materialmischung hineingespritzt wird.

Weitere Merkmale, Vorteile, Eigenschaften und Wirkungen der Erfindung werden nachfolgend anhand von Ausführungsbeispielen und der beigefügten Figuren erläutert. Identische oder äquivalente, insbesondere funktionsgleiche Elemente haben in den Figuren dasselbe Bezugszeichen. Die Bezugszeichen behalten von einer Figur zur anderen ihre Gültigkeit.

Es sei darauf hingewiesen, dass im Rahmen der vorliegenden Beschreibung die Begriffe "aufweisen", "umfassen" oder "beinhalten" keinesfalls das Vorhandensein weiterer Merkmale ausschließen. Ferner schließt die Verwendung des unbestimmten Artikels bei einem Gegenstand nicht dessen Plural aus.

Es zeigen:
- Figur 1:: einen Ausschnitt eines Bauteils in Gestalt eines Elektronikgehäuses einer Pumpenelektronik mit Dichtung in einer Explosionsdarstellung
- Figur 2a-2c:: Beispiele unterschiedlicher Applikationszustände der Materialmischung der Dichtung auf dem Bauteil
- Figur 3:: Vergrößerung des Ausschnitts A aus Figur 1 in einer vereinfachten Darstellung ohne Dichtung
- Figur 4:: Vergrößerung des Ausschnitts A aus Figur 1 in einer vereinfachten Darstellung mit fehlerhafter Dichtung
- Figur 5:: Prinzipdarstellung der Erfassung des Bauteils durch eine Wärmebildkamera
- Figur 6:: beispielhafte thermografische Aufnahme von drei nebeneinanderliegender Bauteile mit applizierter, gegenüber der Umgebung wärmerer Materialmischung
- Figuren 7a, 7b:: Ablaufdiagramm zur Erzeugung eines Referenzdatensatz
- Figuren 8a, 8b:: Ablaufdiagramm zur Anwendung des Referenzdatensatzes

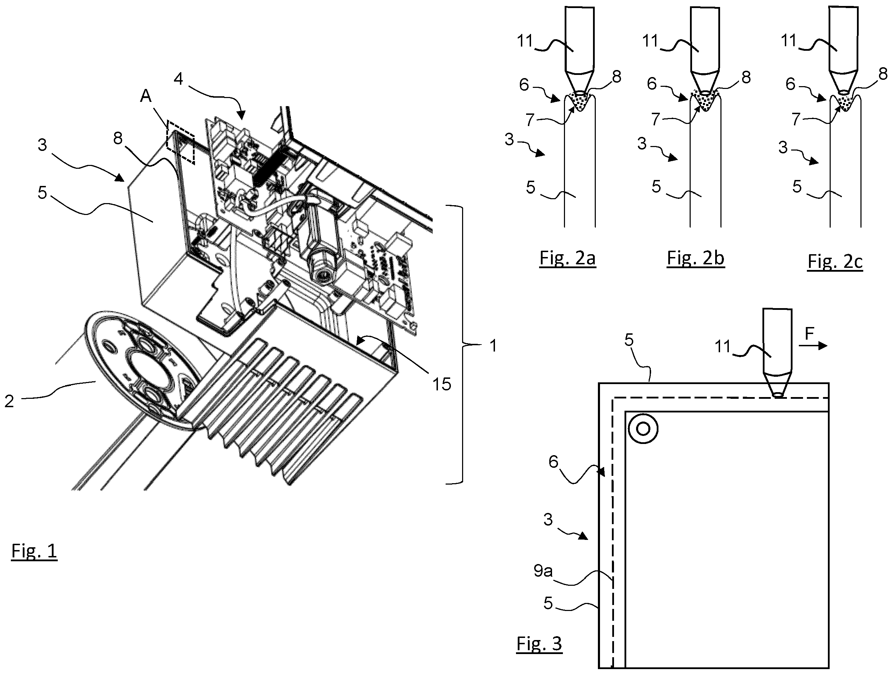

Figur 1 zeigt in der Art einer perspektivischen Explosionsdarstellung einen Ausschnitt einer Pumpenelektronik 4 eines Pumpenaggregats 1. Die Pumpenelektronik 4 ist in im Innenraum 15 eines kastenförmigen Elektronikgehäuses angeordnet, von dem hier nur das Gehäuseunterteil 3 gezeigt ist, das an ein axiales Ende eines Elektromotors 2 montiert ist. Das Gehäuseunterteil 3 bildet ein Bauteil im Sinne der vorliegenden Beschreibung.

Das Elektronikgehäuse 3 weist Seitenwände 5 auf, die an ihren Stirnseiten eine umlaufende Umfangskante 6 mit einer sich längsparallel der Umfangskante 6 erstreckenden Nut 7 besitzen, siehe Figuren 2a-2c. In die Nut 7 ist eine zu einer Dichtung erhärtenden Materialmischung 8 aus einer ersten und einer zweiten Komponente appliziert. Die beiden Komponenten reagieren bei Kontakt miteinander exotherm und schäumen dabei auf, so dass die Dichtung am Ende eine porige Schaumdichtung bildet.

Die Mischung der ersten und zweiten Komponente erfolgt unmittelbar vor oder während des Applizierens in einem änderbar voreingestellten Mischungsverhältnis. Die genaue Voreinstellung des Mischungsverhältnisses ist in der Praxis schwierig und kann sich außerdem im Betrieb ändern, beispielsweise aufgrund sich zusetzender oder verklebender Kanäle, Schläuche oder Ventile, durch die die erste und/ oder zweite Komponente strömt. Zusätzlich ist die Extrusion einer exakten Menge der Materialmischung 8 pro Zeiteinheit durch eine Spritzdüse 11 (siehe Figuren 2a bis 3) problematisch, zumal diese außerdem zeitlich konstant sein soll, um entlang der gesamten umlaufenden Umfangskante 6 dieselbe Menge der Materialmischung 8 zu erhalten. Häufig kommt es dabei zu Fehlern, beispielsweise zu einer zu großen Materialmenge in der Nut, wie Figur 2b zeigt, oder zu einer zu geringen Materialmenge in der Nut 7, wie Figur 2c zeigt. Figur 2a veranschaulicht eine Idealmenge der Materialmischung 8 in der Nut 7. Es sei angemerkt, dass Figuren 2a-2c jeweils einen Querschnitt durch eine Seitenwand 5 des Gehäuseunterteil 3 zeigen.

Figur 3 zeigt die Vergrößerung des Ausschnitts A aus Figur 1 in einer vereinfachten Darstellung und in Draufsicht. Dargestellt ist hier eine Ecke des Gehäuseunterteils 3 samt der beiden sich von dieser weg erstreckenden Seitenwände 5. Die Talsohle der Nut 7 bildet die Mitte eines Soll-Applikationspfades 9a, entlang dem die Applizierung der Materialmischung 8 zu erfolgen hat. Der Soll-Applikationspfades 9a bildet quasi eine Ideallinie, die sich entlang der Umfangskante 6 erstreckt. Die Materialmischung 8 tritt hierzu aus der Spritzdüse 11 heraus, die den Soll-Applikationspfad 9a entlang bewegt wird, siehe Pfeil F.

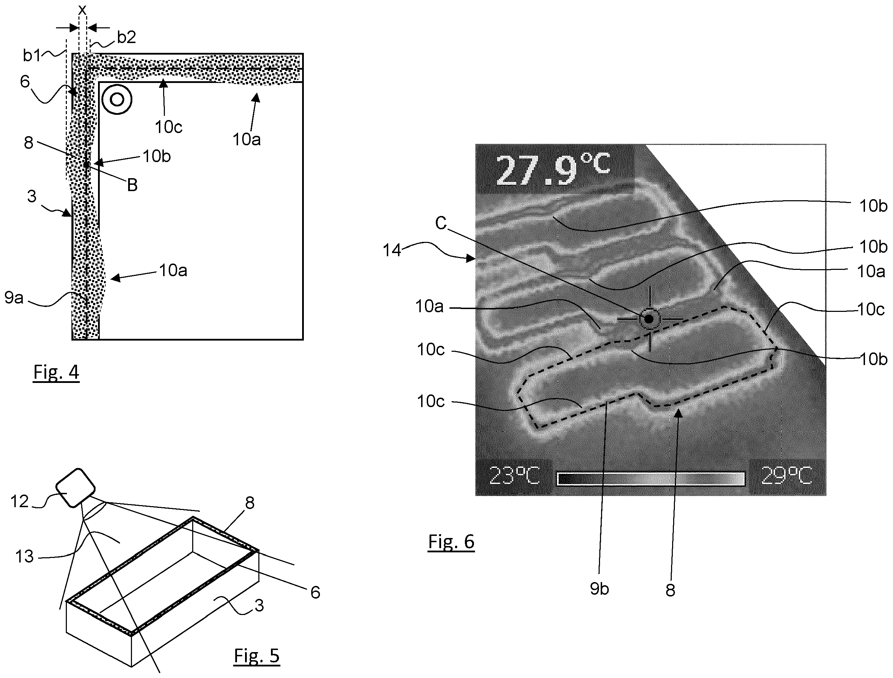

Figur 4 entspricht Figur 3, jedoch mit einer beispielhaft applizierten Materialmischung 8, die entlang des Soll-Applikationspfad 9a einige Fehler aufweist. So gibt es erste Bereiche 10a, in denen das Volumen der Materialmischung 8 zu groß ist, was auf eine zu große Menge der Materialmischung 8 oder auf ein fehlerhaftes, ein zu starkes Aufschäumen bewirkendes Mischungsverhältnis der ersten und zweiten Komponente zurückgeführt werden kann. Ferner gibt es einen zweiten Bereich 10c, in dem das Volumen der Materialmischung 8 zu gering ist, was auf eine zu geringe Menge der Materialmischung 8 oder ebenfalls auf ein fehlerhaftes, ein zu geringes Aufschäumen bewirkendes Mischungsverhältnis der ersten und zweiten Komponente zurückgeführt werden kann. Des Weiteren gibt es einen dritten Bereich 10b, in dem die Materialmischung 8 den Soll-Applikationspfad 9a um ein zu großes Maß x verlässt.

Zur Bestimmung dieses Maßes x kann zunächst die Ausdehnung bzw. Breite der applizierten Materialmischung 8 an einem geometrischen Ort B des Soll-Applikationspfad 9a quer zu diesem bestimmt werden, wobei dies bei der Prüfung der Materialmischung 8 an jedem geometrischen Ort des Soll-Applikationspfad 9a erfolgt. Die Breite ist in Figur 4 durch die beiden Begrenzungslinien b1, b2 veranschaulicht. Daraus kann dann die Mitte der Materialmischung 8 quer zum Soll-Applikationspfad 9a bestimmt werden. Erfolgt diese Bestimmung der Mitte an jedem geometrischen Ort des Soll-Applikationspfad 9a und verbindet man diese geometrischen Mitten miteinander, so erhält man den Verlauf der applizierten Materialmischung 8 und damit dessen Ist-Applikationspfad 9b. Der Abstand der Mitte der Materialmischung 8 zum Soll-Applikationspfad 9a entspricht dem Maß x der Abweichung zwischen dem Ist-Applikationspfad 9b und dem Soll-Applikationspfad 9a am geometrischen Ort B. Übersteigt dieses Maß x einen Grenzwert, liegt ein Fehler vor.

Der erste, zweite und/ oder dritte Bereich 10a, 10b, 10c kann dank der exothermen Reaktion der Komponenten der Materialmischung 8 erkannt werden. Hierzu wird von dem fertigen Bauteil 3, d.h. samt vollständig applizierter Materialmischung 8, mit Hilfe einer Wärmebildkamera 12 eine thermografische Aufnahme 14 erstellt. Figur 5 veranschaulicht dies in einer Prinzipskizze, bei der das Bauteil 3 an dem Gehäuseunterteil 3 in den Figuren 1 bis 4 orientiert ist. Das Bauteil 3 befindet sich hier im Erfassungsbereich 13 der Wärmebildkamera 12, damit diese die thermografische Aufnahme 14 erstellen kann. Es sei angemerkt, dass die Wärmebildkamera 12 in dieser Ausführungsvariante von der Spritzdüse 11 räumlich unabhängig ist.

Figur 6 zeigt eine beispielhafte thermografische Aufnahme 14 von drei nebeneinanderliegenden, jedoch nicht näher erkennbaren Bauteilen 3 jeweils mit kürzlich applizierter Materialmischung 8, so dass deren Komponenten noch immer exotherm reagieren. Während die Umgebung der Bauteile 3 eine Temperatur von 23°C aufweist, liegt die Maximaltemperatur der Materialmischung 8 bei 29°C. Ein Beispielhafter Punkt C der Materialmischung 8, der durch die Mitte des Fadenkreuzes in Figur 6 ausgewiesen ist, hat die Temperatur 27,9°C. Anhand der gemessenen Temperaturen können Fehler in der Art des ersten, zweiten und dritten Bereichs 10a, 10b, 10c in der thermografischen Aufnahme identifiziert werden. Diese Auswertung erfolgt softwarebasiert, beispielsweise indem pixelweise oder pixelgruppenweise die Temperatur der Materialmischung 8 entlang des Applikationspfades 9b bestimmt und geprüft wird, ob wenigstens eine Referenztemperatur über- oder unterschritten ist, und/oder ob der für die Applikation der Materialmischung 8 vorgesehene Soll-Applikationspfad 9a eingehalten wird oder ist.

Alternativ kann die Auswertung mit Hilfe künstlicher Intelligenz (KI) unter Anwendung eines KI-Algorithmusses erfolgen, der eingerichtet ist, die thermografische Aufnahme mit einem Referenzdatensatz zu vergleichen und darauf basierend eine Aussage über den Grad der Übereistimmung oder Abweichung zu diesem zu geben. Der Referenzdatensatz stellt eine Wissensdatenbank dar, die aus mehreren, insbesondere einer Vielzahl thermografischer Aufnahmen von Bauteilen mit fehlerfrei applizierter Materialmischung 8 erstellt worden ist. Der KI-Algorithmus kann außerdem lernfähig sein und seine Wissensdatenbank durch neue Erkenntnisse jederzeit erweitern.

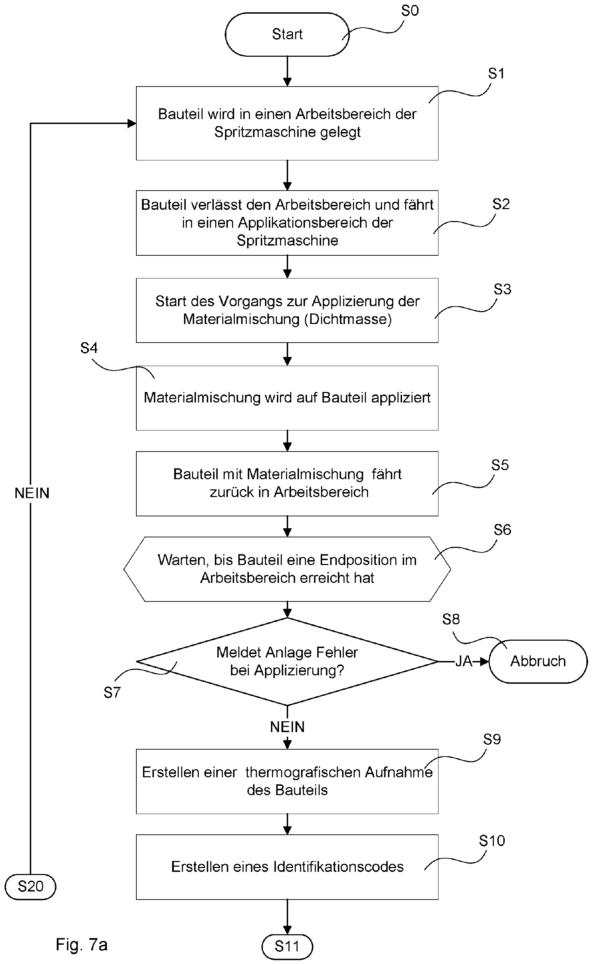

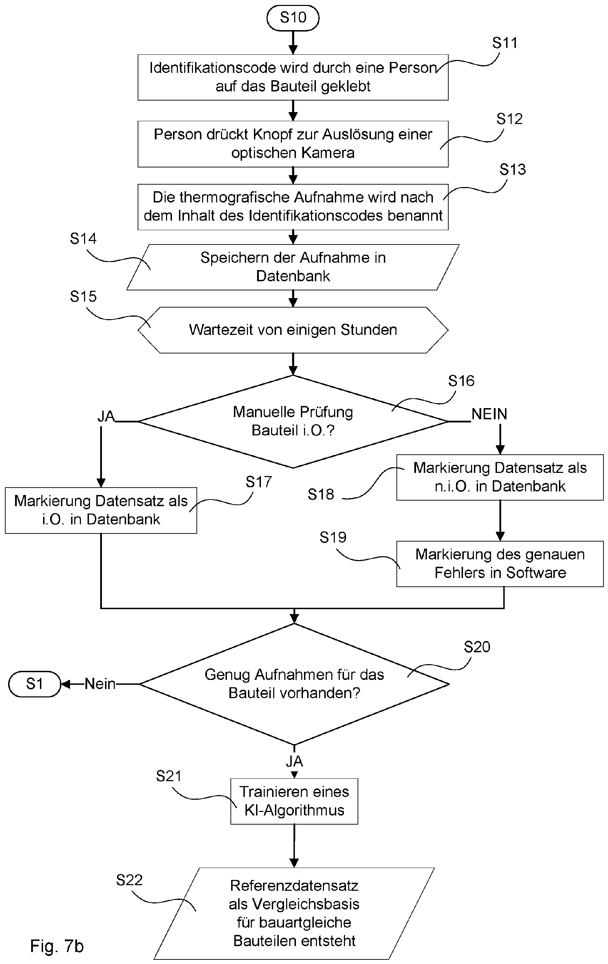

Figuren 7a und 7b zeigen ein Verfahren zur Erzeugung des Referenzdatensatzes. Dies wird nachfolgend erläutert.

Der Startpunkt des Verfahrens ist mit "Start" S0 gekennzeichnet. In einem ersten Schritt S1 wird darauf gewartet, dass ein Bauteil 3, das mit einer Dichtung versehen werden soll, in den Arbeitsbereich einer Spritzmaschine gelegt wird. Das Einlegen erfolgt durch eine die Spritzmaschine bedienende Peron. Anschließend wird dieses Bauteil 3 vom Arbeitsbereich in einen Applikationsbereich der Spritzmaschine transportiert, wobei es den Arbeitsbereich verlässt und in den Applikationsbereich eintritt, Schritt S2. Sodann startet der Vorgang zur Applizierung der Materialmischung 8 (Dichtmasse), Schritt S3, und die Materialmischung 8 wird in eine Nut 7 im Bauteil 3 appliziert, Schritt S4. Schritt S3 umfasst das dosierte Pumpen der beiden Komponenten von jeweils einem Vorratsbehälter zu einer Mischeinrichtung, in der sie in einem bestimmten Verhältnis miteinander gemischt werden, so dass die Materialmischung 8 entsteht. Von der Mischeinrichtung ausgehend wird die Materialmischung 8 zur Spritzdüse 11 gepumpt. Schritt S3 umfasst auch die Positionierung der Spritzdüse 11 an einem Startpunkt am Bauteil, an dem die Applizierung der Materialmischung 8 beginnt.

Die Materialmischung 8 besteht aus einer ersten und einer zweiten Komponente, die während der Schritte S3 und S4 miteinander gemischt werden und dabei exotherm reagieren. Gleichzeitig erfolgt eine Ausgasung, wodurch die Materialmischung 8 aufschäumt und sich in der Nut 7 aufweitet. In Schritt S4 wird die Materialmischung kontinuierlich aus der Spritzdüse 11 herausgepresst, wobei sich die Spritzdüse 11 kontinuierlich den durch den Verlauf der Nut 7 vorgegebenen Soll-Applikationspfad 9a am Bauteil 3 entlang bewegt, bis ein Endpunkt erreicht ist. Dabei kann der Endpunkt dem Startpunkt entsprechen, um eine ringförmig geschlossene Dichtung zu erhalten.

Im nächsten Schritt S5 fährt das Bauteil 3 mitsamt applizierter Materialmischung 8 zurück in den Arbeitsbereich, wobei es in Schritt S6 seine Endposition erreicht hat. Es kann sich ein Schritt S7 anschließen, bei dem geprüft wird, ob die Anlage/ Spritzmaschine während der Applizierung einen Fehler gemeldet hat. Als Fehler kommen hier sämtliche Fehler in Frage, die die Spritzmaschine bei sich und ihrer Peripherie selbständig detektieren kann wie z.B. Motorenstörungen, leere Behälter, Über- oder Untertemperatur, ein Unfall in der Anlage wie z.B. eine Leckage, etc. Ist dies der Fall, wird das Verfahren abgebrochen, Schritt S8. Ist dagegen kein Fehler aufgetreten, wird in Schritt S9 mit Hilfe der Wärmebildkamera 12 eine thermografische Aufnahme 14 von dem Bauteil 3 erstellt bzw. ein Bild aufgenommen.

In einem Folgeschritt S10 wird ein einmaliger Identifikationscode (Bauteil ID) erstellt, der der eindeutigen Identifikation des Bauteils 3 dient. Dies ermöglicht, dass dem Bauteil über diesen Identifikationscode Metadaten zugeordnet werden können, wie z.B. Herstellungszeit- und/ oder Ort und/ oder Prozessparameter wie z.B. ein eingestelltes Mischungsverhält und/oder eine Applikationstemperatur., Ferner kann das Bauteil mit Hilfe des Identifikationscodes in einer Datenbank als "in Ordnung" oder "nicht in Ordnung" vermerkt werden.

Das Verfahren wird bei Schritt S11 in Figur 7b fortgesetzt. In diesem Schritt S11 wird abgewartet, bis eine Person den Identifikationscode manuell auf das Bauteil 3 aufgeklebt hat. Durch den aufgeklebten Identifikationscode kann eine zweite Kamera das Bauteil eindeutig identifizieren. Dies wird genutzt, um das thermographische Foto dem echten, physischen Bauteil zuzuordnen. Diese Zuordnung ist erforderlich, um die Bauteile nach der Applikation zwischenzulagern, zum Beispiel zum Abkühlen, und sie anschließend wieder eindeutig identifizieren zu können.

Anschließend drückt die Person auf einen Knopf zur Auslösung einer zweiten (optischen) Kamera, Schritt S12, um das manuelle Aufbringen des Identifikationscodes zu quittieren und den Identifikationscode auf dem Bauteil zu lesen. Das Bauteil kann dann verknüpft mit dem Identifikationscode als hergestellt in einer Datenbank vermerkt werden. Gegebenenfalls kann auch eine Aufnahme des Bauteils abgespeichert werden. Alternativ zu Schritt S12 kann die Kamera kontinuierlich Bilder aufzeichnen und auswerten und dabei darauf warten, bis sie einen Identifikationscode erkennt. Anschließend kann sie das mit dem Identifikationscode gekennzeichnete Bauteil in der Datenbank abspeichern.

In Schritt S13 wird die thermografische Aufnahme 14 nach dem Inhalt des Identifikationscodes benannt, der beispielsweise aus einer eindeutigen Zahlenabfolge besteht. Mit anderen Worten wird die Datei der thermographischen Aufnahme nach genau dieser Abfolge benannt. Über den Identifikationscode ist somit eine eindeutige Zuordnung des physischen Bauteils 3 zu der dieses Bauteil 3 zeigenden thermografischen Aufnahme 14 geschaffen.

In Schritt S14 wird die thermografische Aufnahme 14 in einer Datenbank gespeichert und es wird einige Stunden gewartet, so dass die Materialmischung vollständig erhärtet und abkühlt, Schritt S15. Nach der Wartezeit erfolgt eine manuelle Prüfung des Bauteils in visueller und haptischer Hinsicht sowie dessen Klassifizierung, Schritt S16. Es wird überprüft, ob das Bauteil eine defektfrei applizierte Dichtung besitzt, d.h. in Ordnung (i.O.) oder nicht in Ordnung (n.i.O.) ist. Ist das Bauteil 3 in Ordnung, wird die thermografische Aufnahme in Schritt S17 in der Datenbank als "in Ordnung" klassifiziert. Ist das Bauteil 3 nicht in Ordnung, wird die thermografische Aufnahme in Schritt S18 in der Datenbank als "nicht in Ordnung" klassifiziert.

Optional kann anschließend in Schritt S19 zusätzlich eine Kategorisierung des Fehlers bzw. eine Zuordnung der thermografischen Aufnahme zu einem konkreten Fehlertyp erfolgen und diese Zuordnung ebenfalls in der Datenbank hinterlegt werden. Beispielsweise kann unterschieden werden zwischen den folgenden Kategorien von Fehlertypen:
1. ein grundsätzlich falsches Mischungsverhältnis der Komponenten,
2. eine grundsätzlich falsche Dosierung der Materialmischung 8, so dass zu viel oder zu wenig Volumen der Materialmischung 8 entlang des gesamten Applikationspfades 9a vorliegt, oder
3. eine lokal falsche Dosierung der Materialmischung 8, so dass entlang des Applikationspfades 9a abschnittsweise zu viel oder zu wenig Volumen der Materialmischung 8 vorliegt.

Im Anschluss an diese Klassifizierung erfolgt in Schritt S20 eine Prüfung, ob ausreichend thermografische Aufnahmen 14 für das Bauteil 3 vorhanden sind, beispielsweise mindestens 150, und zwar sowohl für die Klasse "in Ordnung" als auch für die Klasse "nicht in Ordnung", wobei letztere auch noch in die o.g. Kategorien unterteilt sein kann und dann auch für jede Kategorie ausreichend Aufnahmen vorhanden sein sollten. Dies ist bei der erstmaligen Ausführung des Verfahrens naturgemäß nicht der Fall, so dass das Verfahren bei Schritt S1 fortgesetzt und die Schritte S1 bis S20 wiederholt werden. Je häufiger das Verfahren wiederholt, desto besser ist das Ergebnis. Zu beachten ist dann bei Schritt S9, dass die neue Aufnahme stets nach derselben Zeit seit Applikationsbeginn oder -ende wie die vorherige Aufnahme oder Aufnahmen erfolgen muss, da anderenfalls keine Vergleichbarkeit der Aufnahmen vorliegt, weil die Materialmischung abkühlt.

Sind ausreichend thermografische Aufnahmen 14 für das Bauteil 3 in der Datenbank vorhanden, wird daraus in Schritt S21 der KI-Algorithmus trainiert. Die Datenbank steht folglich einem KI-Algorithmus zur Verfügung, der aus den Aufnahmen den Referenzdatensatz erstellt. Einfach gesprochen, erfolgt das Training derart, dass Zusammenhänge und Gemeinsamkeiten bzw. mathematische Korrelationen zwischen Aufnahmen von identisch klassifizierten Bauteilen ermittelt werden. Durch dieses Training entsteht der Referenzdatensatz, siehe Schritt S22, der dann als Vergleichsgrundlage zur Prüfung bauartgleicher Bauteile verwendet wird.

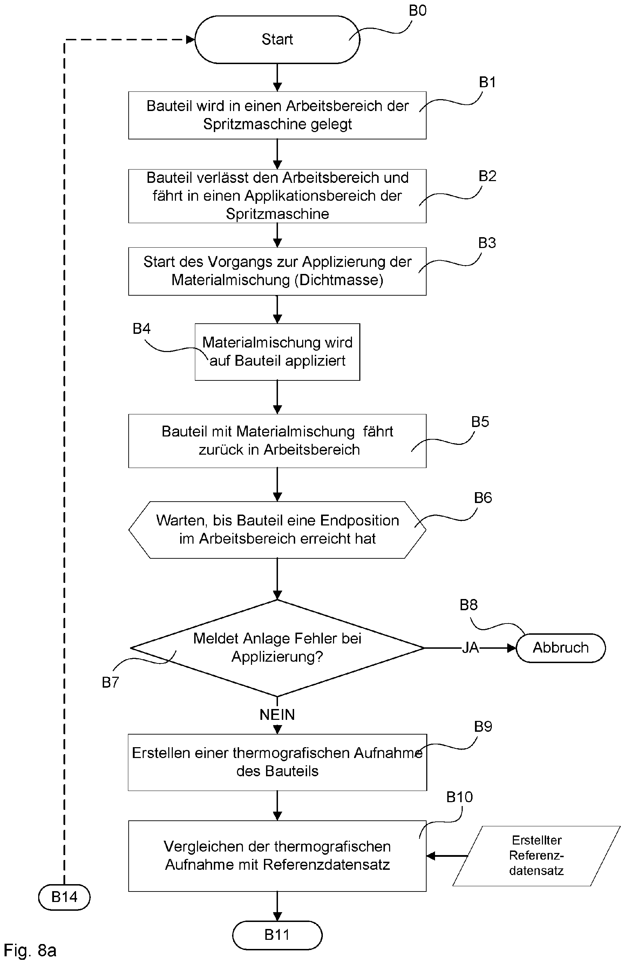

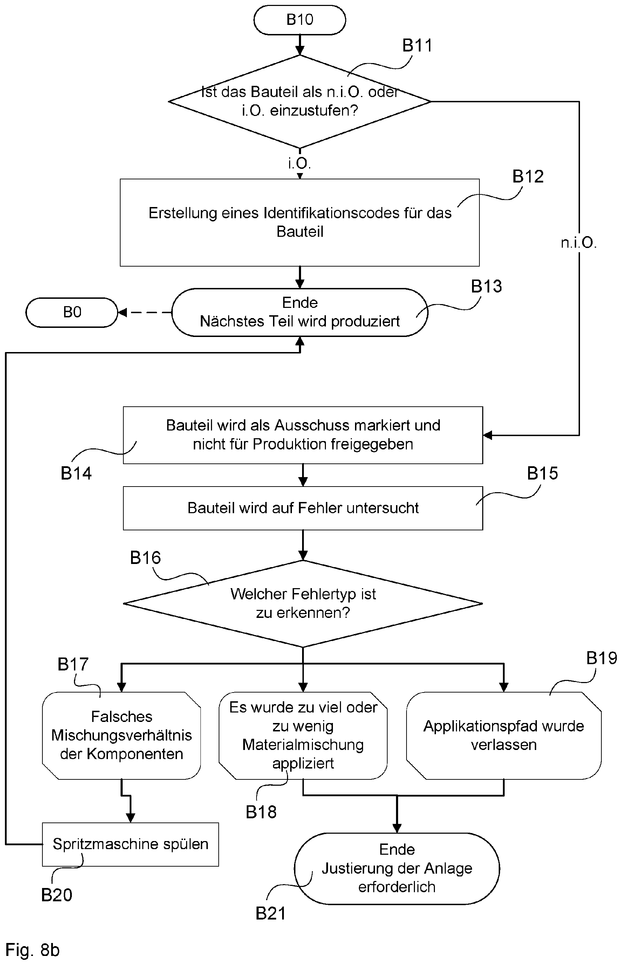

Figuren 8a und 8b zeigen nun ein Verfahren zur Verwendung des Referenzdatensatzes im Rahmen der Prüfung der zu einer Dichtung erhärtenden Materialmischung 8 an einem Bauteil 3. Dies wird nachfolgend erläutert.

Der Startpunkt des Verfahrens ist mit "Start" B0 gekennzeichnet. Die ersten neun Schritte B1 bis B9 sind identisch zu den ersten neun Schritten S1 bis S9 der Figur 7a. So wird in einem ersten Schritt B1 abgewartet, bis ein Bauteil 3, das mit einer Dichtung versehen werden soll, in den Arbeitsbereich der Spritzmaschine eingelegt ist. Anschließend wird dieses Bauteil 3 vom Arbeitsbereich in den Applikationsbereich der Spritzmaschine transportiert, wobei es den Arbeitsbereich verlässt und in den Applikationsbereich eintritt, Schritt B2. Sodann startet der Vorgang zur Applizierung der Materialmischung 8 (Dichtmasse), Schritt B3, und die Materialmischung 8 wird in eine Nut 7 im Bauteil 3 appliziert, Schritt B4. Schritt B3 umfasst das dosierte Pumpen der beiden Komponenten von jeweils einem Vorratsbehälter zu einer Mischeinrichtung, in der sie in einem bestimmten Verhältnis miteinander gemischt werden, so dass die Materialmischung 8 entsteht. Von der Mischeinrichtung ausgehend wird die Materialmischung 8 zur Spritzdüse 11 gepumpt. Schritt B3 umfasst auch die Positionierung der Spritzdüse 11 an einem Startpunkt am Bauteil, an dem die Applizierung der Materialmischung 8 beginnt.

Die Materialmischung 8 besteht auch hier aus einer ersten und einer zweiten Komponente, die während der Schritte B3 und B4 miteinander gemischt werden und dabei exotherm reagieren. Gleichzeitig erfolgt eine Ausgasung, wodurch die Materialmischung 8 aufschäumt und sich in der Nut 7 aufweitet. In Schritt B4 wird die Materialmischung kontinuierlich aus der Spritzdüse 11 herausgepresst, wobei sich die Spritzdüse 11 kontinuierlich den durch den Verlauf der Nut 7 vorgegebenen Soll-Applikationspfad 9a am Bauteil 3 entlang bewegt, bis ein Endpunkt erreicht ist. Dabei kann der Endpunkt dem Startpunkt entsprechen, um eine ringförmig geschlossene Dichtung zu erhalten.

Im nächsten Schritt B5 fährt das Bauteil 3 mitsamt applizierter Materialmischung 8 zurück in den Arbeitsbereich und es wird gewartet, bis das Bauteil dort seine Endposition erreicht hat. Dies ist in Schritt S6 der Fall. Es schließt sich ein Schritt B7 an, bei dem geprüft wird, ob die Spritzmaschine während der Applizierung einen Fehler bei sich oder in seiner Peripherie erkannt hat. Die Fehler sind hier dieselben, wie sie zuvor in Bezug zu Schritt S7 genannt worden sind. Ist ein solcher Fehler erkannt worden, wird das Verfahren abgebrochen, Schritt B8. Anderenfalls (Nein-Zweig) wird in Schritt B9 mit Hilfe der Wärmebildkamera 12 eine thermografische Aufnahme 14 von dem Bauteil 3 erstellt bzw. ein Bild aufgenommen. Ist bei der Applizierung kein Fehler gemeldet worden, löst somit das Erreichen der Endposition die thermografische Aufnahme 14 aus

Diese thermografische Aufnahme 14 bzw. das aufgenommene Bild wird anschließend in Schritt B10 dahingehend ausgewertet, dass die Temperaturen in der Aufnahme, insbesondere der Materialmischung bestimmt und anschließend in Schritt B11 mit dem zuvor erstellen Referenzdatensatz verglichen werden, der an dieser Stelle zur Durchführung dieses Vergleichs zur Verfügung gestellt wird oder für einen Zugriff zur Verfügung steht. Bei dem Vergleich wird analysiert, ob das Bauteil 3 in der thermografischen Aufnahme 14 eher als "in Ordnung" (defektfrei applizierte Materialmischung 8) oder als "nicht in Ordnung" (applizierte Materialmischung weist Defekt/ Fehler auf) zu klassifizieren ist, indem ein Übereinstimmungs- oder Abweichungsgrad der thermografischen Aufnahme zum Referenzdatensatz bestimmt und mit einem Grenzwert verglichen wird. In Schritt B11 wird also der thermografischen Aufnahme 14 respektive dem Bauteil ein "in Ordnung" oder "nicht in Ordnung" Wert zugeordnet.

Ist das Bauteil als "in Ordnung" (i.O.) klassifiziert, wird ein Identifikationscode für das Bauteil erzeugt, beispielsweise eine Seriennummer, Schritt B12. Das Bauteil hat dann die Qualitätskontrolle bestanden. Für eine physische Zuordnung des Identifikationscodes zu dem Bauteil, kann der Identifikationscode auf ein Etikett gedruckt und auf das Bauteil geklebt werden.

Das Verfahren ist sodann beendet., Schritt B13, oder kann mit der Herstellung eines weiteren Bauteils 3 mit einer zu einer Dichtung erhärtenden Materialmischung 8 wiederholt werden. In diesem Fall beginnt das Verfahren erneut beim Start B0.

Ist das Bauteil als "nicht in Ordnung" (n.i.O.) klassifiziert, wird es als Ausschuss markiert, Schritt B14, und somit nicht für die Produktion freigegeben. An dieser Stelle könnte das Verfahren ebenfalls enden oder wiederholt werden.

Es kann jedoch auch eine Fehlertypanalyse erfolgen, siehe Schritt B15, bei der das Bauteil auf den konkreten Fehlertyp hin untersucht wird. Dies kann manuell erfolgen, oder mit Hilfe des KI-Algorithmus. Letzteres erfordert allerdings, dass in der Datenbank, in Schritt S19 von Figur 7b, den einzelnen thermografischen Aufnahmen 14 der "nicht in Ordnung" klassifizierten Bauteilen der Fehlertyp zugeordnet wird, und zu jedem Fehlertyp gemäß Schritt S21 in Figur 7b ein eigener, und zwar fehlertypspezifischer Referenzdatensatz aus den diesem Fehlertyp zugeordneten thermografischen Aufnahmen 14 erstellt worden ist.

Die Fehlertypen können beispielsweise sein:

| | |
|---|---|
| Fehlertyp 1: | ein grundsätzlich falsches Mischungsverhältnis der Komponenten, |
| Fehlertyp 2: | eine grundsätzlich falsche Dosierung der Materialmischung 8, so dass zu viel oder zu wenig Volumen der Materialmischung 8 entlang des gesamten Applikationspfades 9a vorliegt, oder |
| Fehlertyp 3: | ein abschnittsweises Verlassen des Applikationspfads. |

In Schritt B16 wird mittels einer Fallunterscheidung untersucht, welcher Fehlertyp an dem Bauteil 3 zu erkennen ist. Softwaretechnisch kann dies dadurch erfolgen, dass die thermografische Aufnahme 14 des Bauteils 3 nacheinander mit den einzelnen fehlertypspezifischen Referenzdatensätzen verglichen, bei jedem Vergleich jeweils ein Übereinstimmungs- oder Abweichungswert bestimmt, und aus diesen Werten anschließend der größte Übereinstimmungswert oder der kleinste Abweichungswert ermittelt wird, wobei dann jener Fehlertyp angenommen werden kann, dessen fehlertypspezifischer Referenzdatensatz zu dem größten Übereinstimmungswert oder dem kleinsten Abweichungswert führte. In Figur 8b ist Block B17 dem Fehlertyp 1, Block B18 dem Fehlertyp 2 und Block B19 dem Fehlertyp 3 zugeordnet.

Liegt Fehlertyp 1 vor, d.h. ein grundsätzlich falsches Mischungsverhältnis der Komponenten, wird in Reaktion hierauf in Schritt B20 entweder eine elektronische Wartungsnachricht versendet, so dass daraufhin eine Person die Dosiereinheit prüfen kann, oder eine Spülung (Reinigung) der Spritzmaschine durchgeführt, insbesondere automatisch. Durch die Spülung (mit Hochdruck) werden Verunreinigungen und Luftblasen, etc. in den Leitungen der Spritzmaschine beseitigt. Danach wird das Verfahren dann beendet, Schritt B13, bzw. gegebenenfalls wiederholt.

Liegt Fehlertyp 2 oder 3 vor, d.h. zu viel oder zu wenig applizierte Materialmischung 8 oder ein Verlassen des Applikationspfads 9a, muss die Spritzmaschine neu justiert werden, bevor das Verfahren mit einem neuen Bauteil 3 wiederholt werden kann. Hierüber kann eine entsprechende Meldung ausgegeben werden. Das Verfahren ist dann beendet, Schritt B21. Allerdings kann auch bei diesen Fehlerbildern eine automatische Einleitung von Maßnahmen zur Fehlerbehebung erfolgen.

Es sei darauf hingewiesen, dass die vorstehende Beschreibung lediglich beispielhaft zum Zwecke der Veranschaulichung gegeben ist und den Schutzbereich der Erfindung keineswegs einschränkt. Merkmale der Erfindung, die als "kann", "beispielhaft", "bevorzugt", "optional", "ideal", "vorteilhaft", "gegebenenfalls" oder "geeignet" angegeben sind, sind als rein fakultativ zu betrachten und schränken ebenfalls den Schutzbereich nicht ein, welcher ausschließlich durch die Ansprüche festgelegt ist. Soweit in der vorstehenden Beschreibung Elemente, Komponenten, Verfahrensschritte, Werte oder Informationen genannt sind, die bekannte, naheliegende oder vorhersehbare Äquivalente besitzen, werden diese Äquivalente von der Erfindung mit umfasst. Ebenso schließt die Erfindung jegliche Änderungen, Abwandlungen oder Modifikationen von Ausführungsbeispielen ein, die den Austausch, die Hinzunahme, die Änderung oder das Weglassen von Elementen, Komponenten, Verfahrensschritte, Werten oder Informationen zum Gegenstand haben, solange der erfindungsgemäße Grundgedanke erhalten bleibt, ungeachtet dessen, ob die Änderung, Abwandlung oder Modifikationen zu einer Verbesserung oder Verschlechterung einer Ausführungsform führt.

Obgleich die vorstehende Erfindungsbeschreibung eine Vielzahl körperlicher, unkörperlicher oder verfahrensgegenständlicher Merkmale in Bezug zu einem oder mehreren konkreten Ausführungsbeispiel(en) nennt, so können diese Merkmale auch isoliert von dem konkreten Ausführungsbeispiel verwendet werden, jedenfalls soweit sie nicht das zwingende Vorhandensein weiterer Merkmale erfordern. Umgekehrt können diese in Bezug zu einem oder mehreren konkreten Ausführungsbeispiel(en) genannten Merkmale beliebig miteinander sowie mit weiteren offenbarten oder nicht offenbarten Merkmalen von gezeigten oder nicht gezeigten Ausführungsbeispielen kombiniert werden, jedenfalls soweit sich die Merkmale nicht gegenseitig ausschließen oder zu technischen Unvereinbarkeiten führen.

## Patentansprüche

1. Verfahren zur softwarebasierten Prüfung einer entlang eines Applikationspfades (9b) spritztechnisch an einem Bauteil (3), insbesondere an einem Elektronikgehäuse eines Pumpenaggregats, applizierten und zu einer Dichtung erhärtenden Materialmischung (8) aus wenigstens einer ersten und einer zweiten Komponente, die unmittelbar vor oder während des Applizierens in einem änderbar voreingestellten Mischungsverhältnis gemischt werden und dabei exotherm miteinander reagieren, **dadurch gekennzeichnet, dass** während der exothermen Reaktion mit einer Wärmebildkamera (12) wenigstens eine thermografische Aufnahme (14) der applizierten Materialmischung (8) erstellt und anschließend softwarebasiert daraufhin ausgewertet wird, ob die Temperatur der Materialmischung (8) entlang des Applikationspfades (9b) wenigstens eine Referenztemperatur über- oder unterschreitet, und/oder ob ein für die Applikation der Materialmischung vorgesehener Soll-Applikationspfad (9a) eingehalten wird oder ist.

2. Verfahren nach Anspruch 1, **dadurch gekennzeichnet, dass** die thermografische Aufnahme(n) (14) mit wenigstens einem aus Referenzbildern von Bauteilen mit fehlerhaft und/ oder von Bauteilen mit fehlerfrei applizierter Materialmischung (8) erstellten Referenzdatensatz verglichen wird.

3. Verfahren nach Anspruch 2, **dadurch gekennzeichnet, dass** ein Übereinstimmungs- oder Abweichungsgrad der thermografischen Aufnahme(n) (14) zum Referenzdatensatz bestimmt wird, und eine Meldung ausgegeben, die Applikation der Materialmischung (8) beendet und/ oder eine Korrekturmaßnahme eingeleitet wird, wenn der Übereinstimmungsgrad einen Grenzwert unterschreitet oder der Abweichungsgrad einen Grenzwert überschreitet.

4. Verfahren nach Anspruch 1, **dadurch gekennzeichnet, dass** aus der thermografischen Aufnahme (14) der Materialmischung (8) entlang des Applikationspfades (9a) zuordenbare, lokal maximale Temperaturwerte bestimmt werden, die jeweils mit der wenigstens einen Referenztemperatur verglichen werden, wobei eine Meldung ausgegeben, die Applikation der Materialmischung beendet und/ oder eine Korrekturmaßnahme eingeleitet wird, wenn die Referenztemperatur an wenigstens einer Stelle des Applikationspfades (9a) nicht erreicht oder überschritten wird.

5. Verfahren nach Anspruch 4, **dadurch gekennzeichnet, dass** die Referenztemperatur eine Mindesttemperatur ist und die Meldung ausgegeben, die Applikation beendet und/ oder die Korrekturmaßnahme eingeleitet wird, wenn die Referenztemperatur nicht erreicht wird.

6. Verfahren nach Anspruch 4 oder 5, **dadurch gekennzeichnet, dass** die Referenztemperatur oder eine zweite Referenztemperatur eine Maximaltemperatur ist und die Meldung ausgegeben, die Applikation beendet und/ oder die Korrekturmaßnahme eingeleitet wird, wenn die Referenztemperatur erreicht oder überschritten wird.

7. Verfahren nach einem der vorherigen Ansprüche, **dadurch gekennzeichnet, dass** aus lokal maximalen Temperaturwerten die Lage und/ oder der Verlauf der Materialmischung (8) innerhalb der thermografischen Aufnahme (14), insbesondere ein Ist-Applikationspfad (9b), entlang welchem die lokal maximalen Temperaturwerten liegen, bestimmt wird, und dass die Lage und/ oder der Verlauf der Materialmischung, insbesondere der Ist-Applikationspfad (9b), mit dem Soll-Applikationspfad (9a) verglichen wird, wobei eine Meldung ausgegeben, die Applikation beendet und/ oder die Korrekturmaßnahme eingeleitet wird, wenn die Lage und/ oder der Verlauf der Materialmischung (8), insbesondere der Ist-Applikationspfad (9b), an wenigstens einer Stelle um ein vorgegebenes Maß vom Soll-Applikationspfad (9a) abweicht.

8. Verfahren nach einem der vorherigen Ansprüche, **dadurch gekennzeichnet, dass** die Wärmebildkamera (12) die Materialmischung (8) an einem ersten Ort des Soll-Applikationspfades (9a) erfasst, während die Materialmischung (8) an einem zweiten Ort des Soll-Applikationspfades (9a) an dem Bauteil (3) appliziert wird.

9. Verfahren nach einem der vorherigen Ansprüche, **dadurch gekennzeichnet, dass** die Wärmebildkamera (12) das gesamte Bauteil (3) nach der Applikation der Materialmischung (8) erfasst.

10. Verfahren zumindest nach Anspruch 2, **dadurch gekennzeichnet, dass** das Bauteil (3) einer Serie von Bauteilen entstammt, denen auf derselben Spritzmaschine die Materialmischung (8) appliziert worden ist, wobei der Referenzdatensatz aus thermografischen Aufnahmen (14) der ersten hergestellten Bauteile mit applizierter Materialmischung (8) der Serie erstellt ist.

11. Verfahren zumindest nach Anspruch 2, **dadurch gekennzeichnet, dass** die thermografische Aufnahme (14) der applizierten Materialmischung (8) und die thermografischen Aufnahmen (14), aus denen der Referenzdatensatz erstellt wurde, zu im Wesentlichen gleichen Zeitpunkten nach der vollständigen Applizierung der Materialmischung (8) auf das jeweilige Bauteil (3) aufgenommen worden sind.
